(19) Europäisches Patentamt
European Patent Office
Office européen des brevets

(11) **EP 0 924 519 B1**

(12) **EUROPÄISCHE PATENTSCHRIFT**

(45) Veröffentlichungstag und Bekanntmachung des Hinweises auf die Patenterteilung:
**04.01.2006 Patentblatt 2006/01**

(51) Int Cl.:
*G01N 33/48* (2006.01)      *G01N 33/86* (2006.01)
*G06F 19/00* (2006.01)      *A61K 38/58* (2006.01)

(21) Anmeldenummer: **98121054.5**

(22) Anmeldetag: **06.11.1998**

(54) **Verfahren zur Ermittlung eines Dosierungsschemas für Thrombininhibitoren**

Process for the determination of a dosing regimen for thrombin inhibitors

Procédé de détermination d'un régime posologique pour inhibiteurs de thrombine

(84) Benannte Vertragsstaaten:
**CH DE ES FR GB IT LI**

(30) Priorität: **15.12.1997 DE 19755682**

(43) Veröffentlichungstag der Anmeldung:
**23.06.1999 Patentblatt 1999/25**

(73) Patentinhaber: **Abbott GmbH & Co. KG**
**65205 Wiesbaden (DE)**

(72) Erfinder:
- **Nörtersheuser, Peter Dr.**
  **55127 Mainz (DE)**
- **Kreis, Siegfried**
  **67251 Freinsheim (DE)**

(74) Vertreter: **Grünecker, Kinkeldey,**
**Stockmair & Schwanhäusser**
**Anwaltssozietät**
**Maximilianstrasse 58**
**80538 München (DE)**

(56) Entgegenhaltungen:
**WO-A-93/16390**

- **POETZSCH B ET AL: "MONITORING OF RECOMBINANT HIRUDIN: ASSESSMENT OF A PLASMA-BASED ECARIN CLOTTING TIME ASSAY" THROMBOSIS RESEARCH,TARRYTOWN, NY,US, Bd. 86, Nr. 5, 1. Juni 1997 (1997-06-01), Seiten 373-383, XP001000734 ISSN: 0049-3848**
- **WAKEFIELD J ET AL: "AN APPLICATION OF BAYESIAN POPULATION PHARMACOKINETIC/PHARMACODYNAMIC MODELS TO DOSE RECOMMENDATION" STATISTICS IN MEDICINE, WILEY, NEW YORK, NY, US, Bd. 14, Nr. 9/10, Mai 1995 (1995-05), Seiten 971-986, XP001008622 ISSN: 0277-6715**
- **MUNGALL D R ET AL: "A PROSPECTIVE RANDOMIZED COMPARISON OF THE ACCURACY OF COMPUTER-ASSISTED VERSUS GUSTO NOMOGRAM-DIRECTED HEPARIN THERAPY" CLINICAL PHARMACOLOGY & THERAPEUTICS, MOSBY-YEAR BOOK, ST LOUIS, MO, US, Bd. 55, Mai 1994 (1994-05), Seiten 591-596, XP001008447 ISSN: 0009-9236**
- **GOMENI R ET AL: "POPULATION KINETICS AND CONDITIONAL ASSESSMENT OF THE OPTIMAL DOSAGE REGIMEN USING THE P-PHARM SOFTWARE PACKAGE" ANTICANCER RESEARCH, HELENIC ANTICANCER INSTITUTE, ATHENS,, GR, Bd. 14, 8. November 1994 (1994-11-08), Seiten 2321-2326, XP001008384 ISSN: 0250-7005**

**Beschreibung**

Die Erfindung betrifft ein Verfahren zur Ermittlung eines Dosierungsschemas für Hirudine und/oder oralen Thrombininhibitoren.

Thrombin spricht als proteolytisches Enzym eine wesentliche Rolle bei der Blutkoagulation. Thrombininhibitoren sind daher interessante Wirkstoffe bei der Behandlung von Krankheiten wie der Thrombose. Als Thrombininhibitoren sind insbesondere Heparine, aber auch Hirudin bzw. Hirudinderivate sowie orale Thrombininhibitoren bekannt, wobei es für die Wirksamkeit dieser Thrombininhibitoren im wesentlichen auf die Blut- bzw. Plasmaspiegel dieser Verbindungen ankommt.

In der WO 93/16390 ist ein Verfahren zur Bestimmung von Hirudin und synthetischen Thrombininhibitoren im Blut beschrieben, gemäß welchem zu dem Blut oder dem Blutbestandteil ein Prothrombin-Intermediat, eine Verbindung, die Prothrombin zu Meizothrombin spaltet, ein Salz davon oder ein Gemisch dieser Verbindungen sowie gegebenenfalls Puffer und/oder andere übliche Zusatzstoffe gegeben werden und die Zeit, die von der Zugabe bis zum Beginn der Gerinnung vergeht, gemessen wird.

Als Verbindung, die Prothrombin zu Meizothrombin spaltet, wird bevorzugt das Schlangengift Ecarin verwendet. Dieses Verfahren wird daher im folgenden Ecarin-Clotting-Test ("ECT") genannt. Die Gerinnung wird dabei durch Ecarin eingeleitet, es bilden sich die Intermediate, die durch Hirudin oder andere Thrombininhibitoren (nicht durch Heparin) abgefangen werden. Nach einer bestimmten Zeit, wenn das im Blut vorhandene Hirudin oder die anderen Thrombininhibitoren verbraucht sind, gerinnt die Blutprobe. Die Menge Thrombininhibitor im Blut ist aus der Zeit bis zur Gerinnung durch Eichkurven bestimmbar. Die WO 93/16390 macht aber keine Aussage zur Frage, wie die Konzentration eines Thrombininhibitors im Blut eines Patienten vorhergesagt und somit optimal eingestellt werden kann.

In Clin. Pharmacokinet. 1997 Feb. 32 (2), 145 - 172 wird die pharmakokinetische Optimierung bei der Behandlung von tiefen Venenthrombosen beschrieben, insbesondere bei der Behandlung mit Heparin, wobei auf die Notwendigkeit des individuellen Monitoring der Dosierung und die Schwierigkeiten bei nichtlinearen Pharmakokinetiken hingewiesen wird.

Aufgrund der hohen inter- und intra-individuellen Variabilität von Heparin oder anderen Antikoagulantien (z.B. auch PEG-Hirudin) ist ein intensives Monitoring und eine individualisierte Therapie nötig, um das individuelle Nutzen/Risiko-Verhältnis der Behandlung zu optimieren. Probleme bei der Behandlung mit Heparin sind z.B.:

- Nicht adäquate initiale Dosis von Heparin,

- lange Analysezeiten im Labor, die eine schnelle Dosisadjustierung von Heparin verhindern,

- falsche Dosisadjustierung.

Die Verwendung des Ecarin-Clotting-Tests zur Bestimmung von rekombinantem Hirudin und die Vorteile dieses Tests werden beschrieben in *Thrombosis Research.* Vol. 86, Nr. 5, S. 373-383, 1997.

Ein pharmakokinetisches/pharmakodynamisches Bayes'sches Populationsmodell und dessen Verwendung zur Bestimmung einer Dosisempfehlung werden in *Statistics in Medicine,* Vol. 14, 971-986 (1985) dargestellt.

Ein Verfahren zur Ermittlung eines Dosierungsschemas für Heparin unter Verwendung eines Computerprogramms auf Basis eines pharmakokinetischen/pharmakodynamischen Modells wird in *Clinical Pharmacology & Therapeutics,* Mosby-Year Book, St. Louis, Mo, US, Bd. 55, Mai 1994, S. 591-596 beschrieben.

Aufgabe der vorliegenden Erfindung ist es, ein Verfahren zur Verfügung zu stellen, mit dem in einfacher und schneller Weise ein für den einzelnen Patienten optimales Dosierungsschema für Hirudine oder orale Thrombininhibitoren ermittelt werden kann, so dass das Risiko einer Überdosierung mit der Gefahr einer Blutung oder einer Unterdosierung mit der Gefahr einer Thrombosierung deutlich reduziert bzw. eliminiert wird.

Die Aufgabe wird gelöst durch ein Verfahren zur Ermittlung eines Dosierungsschemas für Hirudine oder orale Thrombininhibitoren, dadurch gekennzeichnet, dass man

a) nach Zugabe einer Standarddosis des Hirudins oder des oralen Thrombininhibitors im Blut des Empfängers die Konzentration des Hirudins oder des oralen Thrombininhibitors mit dem Ecarin-Clotting-Time-Test bestimmt und

b) aus dem Verhältnis von in Schritt a) zugegebener Dosis zu der ermittelten Ecarin-Clotting-Time mittels eines Computer-Programms, z.B. das Statistikprogrammpaket p-PHARM 1.4, SIMED, 1996, auf Basis eines pharmakokinetischen / pharmakodynamischen Modells, das durch die folgende Gleichung (6) dargestellt wird,

$$C_{ss\_i} = \frac{D\_i \times f\_i}{V\_i \times k_{el\_i} \times \tau\_i} \quad : \text{(subkutane Injektion)} \quad (6)$$

wobei

$C_{ss\_i}$ die individuelle 'steady state' Inhibitor Plasma Konzentration [ng/ml] ist;

$D\_i$ die verabreichte Dosis [mg] im Applikationsintervall ist;

$f\_i$ die Bioverfügbarkeit der subkutanen Administration [ohne Dimension] ist;

$V\_i$ das scheinbare Verteilungsvolumen [l] von Hirudin im Patienten ist;

$k_{el\_}i$ die scheinbare Eliminationsrate [1/h] von Hirudin im Patienten ist; und

$\tau\_i$ das Applikationsintervall [h] ist,

die Dosierung des weiterhin zuzugebenden Hirudins oder oralen Thrombininhibitors ermittelt.

Mit den erfindungsgemäßen Verfahren wird es in überraschend einfacher Weise möglich, optimale Dosierungsschematas individuell für einzelne Patienten zu entwickeln, wobei das Risiko einer Unterdosierung (mit der Gefahr einer nicht ausreichenden Thrombinhemmung) ebenso minimiert werden kann wie insbesondere das Risiko einer Überdosierung (mit der Gefahr von Blutungen).

Es hat sich herausgestellt, daß die Verwendung der Ecarin-Zeit-Methode (ECT) in Verbindung mit den entsprechenden Patientendaten beim erfindungsgemäßen Verfahren in ausgezeichneter Weise Aussagen insbesondere zur Wahrscheinlichkeit von Blutungen bei der Behandlung mit Hirudinen und/oder oralen Thrombininhibitoren zuläßt.

Das erfindungsgemäße Verfahren hat u.a. folgende Vorteile:

- Durchführung von Modellsimulationen als unterstützende Maßnahme bei der Planung klinischer Studien und Monitoring von Patienten,

- Identifizierung und Quantifizierung eventuell vorhandener Abhängigkeiten der Pharmakokinetik/Pharmakodynamik (PK / PD) von individuellen Einflußfaktoren (z.B. Alter, Geschlecht, renales Exkretionsvermögen, Begleitmedikation) zur Erklärung der Variabilität der beobachteten Arzneimittelwirkung innerhalb klinischer Studien,

- Reduzierung der benötigten Laboranalysen durch konsequentes Anwenden der Möglichkeiten der 'sparse data analysis' Methoden,

- Beschleunigung der klinischen Entwicklung,

- Identifikation von Risikofaktoren bzw. Risikopatienten.

Als Hirudine werden gemäß dem vorliegenden Verfahren bevorzugt die natürlichen Hirudine, aber auch rekombinante Hirudine oder an organische Träger kovalent gebundene Hirudine eingesetzt, wie sie z.B. in der EP 209 061, in der US 4 745 177, in der EP 345 616, in der EP 502 962 oder in der DE 38 05 540 beschrieben werden. Besonders bevorzugt wird an Polyethylenglykol gebundenes Hirudin (EP 502 962 A) und rekombinantes Hirudin wie in der EP 345 616 angegeben eingesetzt.

Als orale Thrombininhibitoren sind niedermolekulare organische Verbindungen verwendbar, die die erforderliche orale Verfügbarkeit aufweisen. Derartige orale Thrombininhibitoren sind z.B. in der WO 94/29336, der EP 669 317, der WO 95/23609, oder der WO 96/25426 beschrieben, worauf ausdrücklich Bezug genommen wird. Insbesondere werden orale Thrombininhibitoren eingesetzt, die als Struktureinheit einen Phenyl- oder heterocyclischen Ring mit einer Amidingruppe als Substituent aufweisen.

Die Bestimmung der Ecarin-Clotting-Time wird gemäß der in der WO 93/16390 beschriebenen Methode durchgeführt.

In Verbindung mit den Daten des Empfängers, d.h. des zu behandelnden Patienten, insbesondere des Geschlechts, des Körpergewichts, des Alters und der Nierenfunktion bzw. des renalen Exkretionsvermögens läßt sich aus dem Verhältnis der zugegebenen primären Standarddosis zu der gemessenen Ecarin-Clotting-Time (oder "Ecarin-Gerinnungszeit"), die mit dem erreichten Blutplasmaspiegel des Thrombininhibitors eine hohe lineare Korrelation aufweist, mittels eines populationsbasierten pharmakokinetisch/pharmakodynamischen (pk/pd) Modelles ein Dosierungsschema ermitteln, aus dem insbesondere hervorgeht, welche Blutungswahrscheinlichkeiten in Abhängigkeit von weiteren Dosierungen des Thrombininhibitors bestehen bzw. ob eine Unterdosierung mit der Gefahr einer Thrombosierung auftreten kann.

Das erfindungsgemäße Verfahren ist unabhängig von der Anwendungsform einsetzbar, d.h. die Thrombininhibitoren können als Infusion oder i.v. Bolus oder als subkutane Injektion oder auch oral verabreicht werden. Es sind auch kombinierte Anwendungsformen möglich und bei bestimmten Indikationen z.B. instabiler Angina pectoris oder peripherer arterieller Verschlußkrankheit sogar bevorzugt.

Das Dosierungsschema wird beim erfindungsgemäßen Verfahren wie folgt ermittelt:

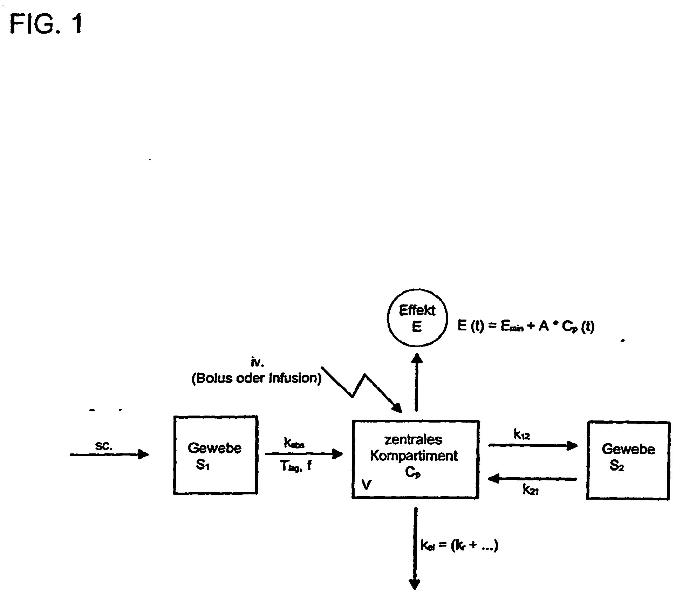

Basierend auf Untersuchungen zur Pharmakokinetik und Pharmakodynamik von PEG-Hirudin (auf der Basis der Daten von mehr als 300 Menschen) wurde ein mathematisches Modell (Abbildung 1) entwikkelt, daß zur Vorhersage und zur Simulation des Zeitverlaufes der PEG-Hirudin Konzentration im Plasma sowie der "Ecarin-Clotting-Time" bei verschiedenen Administrationswegen geeignet ist. Es ermöglicht z.B. aufgrund von demographischen Daten des Patienten (im wesentlichen Alter, Geschlecht, Gewicht, Raucher/Nichtraucher, Nierenfunktion) eine 'a priori' Dosisempfehlung zu geben.

Für PEG-Hirudin bzw. den entsprechenden Thrombininhibitor wird jedoch als noch wesentlicher angesehen, aufgrund einer frühen Inhibitor-Konzentration- oder Ecarin-Clotting-Time-Bestimmung verläßliche Prognosen über die individuelle Sensitivität und den weiteren Konzentrationsverlauf zu geben. Mit dieser frühen Messung lassen sich 'steady state' Konzentrationen prognostizieren und frühzeitig eventuell notwendige Dosiskorrekturen vornehmen.

In Abb. 1 haben die Abkürzungen folgende Bedeutung:

$V$ :    scheinbares Verteilungsvolumen [1]

$k_{el}$:    scheinbare Eliminationsrate [1/h], faßt alle Prozesse zusammen, die zum Verschwinden von PEG-Hirudin aus dem System beitragen (z.B. Metabolismus, Renale Exkretion von PEG-Hirudin ($k_r$))

$Cl_{tot}$:    totale Plasma-Clearance [1/h], berechnet mit kel * V

$k_{12}, k_{21}$:    Transferraten [1/h]

$k_{abs}$:    Absorptionsrate [1/h]

$T_{lag}$:    Zeitverzögerung bei der Absorption [h]

$f$:    Bioverfügbarkeit der subkutanen Administration [ohne Dimension]

$E_{min}$:    Beobachtete 'Ecarin Gerinnungszeit' ohne Einwirkung von PEG-Hirudin (s)

$A$:    Proportionalitätsfaktor [ml * s/ng]

$E(t)$:    'Ecarin Gerinnungszeit' zum Zeitpunkt t (s)

$Cp(t)$:    PEG-Hirudin Konzentration im zentralen Kompartiment zum Zeitpunkt 't' [ng/ml]

$S_1$:    PEG-Hirudin Konzentration am Ort der subkutanen Injektion [ng/ml]

$S2$:    PEG-Hirudin Konzentration im peripheren Kompartiment [ng/l]

iv.:    intravenöse Administration

sc.:    subkutane Administration

Die Modellfunktionen zur Prognose des Zeitverlaufes der Inhibitor-Konzentration und Ecarin Verklumpungszeit für einen bestimmten Patienten, gekennzeichnet durch den Index i kann wie folgt dargestellt werden:

$$\frac{dS_{1\_i}}{dt} = - k_{abs\_i} \cdot S_{1\_i} \tag{1}$$

$$\frac{dCp\_i}{dt} = k_{abs\_i} \cdot S_{1\_i} - \left[ \frac{Cl\_i}{V\_i} + k_{12\_i} \right] \cdot Cp\_i + k_{21\_i} \cdot S_{2\_i} \tag{2}$$

$$\frac{dS_{2\_i}}{dt} = - k_{12\_i} \cdot Cp\_i - k_{21\_i} \cdot S_{2\_i} \tag{3}$$

$$E(t) = E_{min} + A \cdot Cp(t)$$

$$E\_i(t) = E_{min\_i} + A\_i \cdot Cp\_i(t) \tag{4}$$

Basierend auf den ermittelten Werten für die Modellparameter können folgende sekundäre Variablen näherungsweise berechnet werden, auf deren Basis letztendlich eine eventuell notwendige Dosiskorrektur vorgenommen wird

$C_{ss\_i}$:                individuelle 'steady state' Inhibitor Plasma Konzentration (ng/ml)

$$C_{ss\_i} = \frac{R^{\circ}\_i}{V\_i \cdot k_{el\_i}} \qquad : \text{(Infusion)} \tag{5}$$

$$C_{ss\_i} = \frac{D\_i \cdot f\_i}{V\_i \cdot k_{el\_i} \cdot \tau\_i} \quad : \text{(subkutane Injektion)} \tag{6}$$

$E_{ss\_i}$:                individuelle 'steady state Ecarin Verklumpungszeit (s)

$$E_{ss}\_i = E_{min\_i} + A \cdot C_{ss\_i} \tag{7}$$

wobei D:      verabreichte Dosis im Applikationsintervall [mg]
R°:      Infusionsrate [mg/h]
τ:       Applikationsintervall [h]

Die interindividuelle Variabilität hinsichtlich der Exposition mit dem Inhibitor (z.B. PEG-Hirudin) bzw. des Ecarin Zeitverlaufs wird mathematisch beschrieben durch die individuelle Ausprägung der Modellparameter (sogenannte feste Effekte). Dabei wird eine lognormale Verteilung für diejenigen Modellparameter angenommen, welche die Annahme eine rechtsschiefen Verteilung motivieren ($k_{abs}$, $k_{12}$, $k_{21}$). Die logarithmierten Werte dieser Modellparameter sind normal verteilt mit dem Mittelwert (und der Varianz-Kovarianz Matrix C (SIMED, 1996, P-PHARM, A Population Pharmacokinetic-Dynamic data Modeling Program, Version 1.4. SIMED, 94008 Creteil France). Für die anderen Modellparameter (V, C1, Emin, f) wird Normalverteilung angenommen.
Es wird weiter angenommen, daß die unerklärbaren inter- und intra-individuellen Effekte (zufällige Effekte) proportional zum beobachteten Meßwert zunehmen (heteroskedastischer Meßfehler).
Die statistische Auswertung wird wie folgt durchgeführt:
Die Bestimmung der populations- und individuellen Bayes'schen pk/pd Parameter wurde durchgeführt mit dem Statistikprogrammpaket P-PHARM 1.4 (SIMED, 1996), basierend auf dem EM Algorithmus, zuerst beschrieben bei Dempster et al. (Dempster, A.P., N.M. Laird, and D.B Rubin, 1977. Maximum likelihood from incomplete data via the EM algorithm. Journal of the Royal Statistical Society, Series B, 39, 1 - 38). Mathematische Details des implementierten Algorithmus werden ausführlich im Begleitbuch von P-PHARM (SIMED, 1996), bzw. bei Bresolle et al. (Bresolle F., P. Ray, J. M. Jacquet, J. Bres et al., 1991. Cancer Chemother.

Pharmacol., 29: 53 - 60), Fabre et al. (Fabre, D. Bresolle, F. R. Gomeni, O. Bouvet et al., 1993. Clin. Pharmacokinet., 24: 333 - 343) oder Gomeni et al. (Gomeni R., G. Pineau, F. Mentre (1994). Population kinetics and conditional assessment of the optimal dosage regimen using the P-PHARM software package. Anticancer Res. 14: 2321 - 2326) behandelt.

Die weitere statistische Analyse der abgeleiteten pk/pd Parameter und die Identifikation der Kovariablen wurde mit dem Statistikprogramm SAS (Version 6.12, Cary, NC: SAS Institute Inc., 1996) durchgeführt.

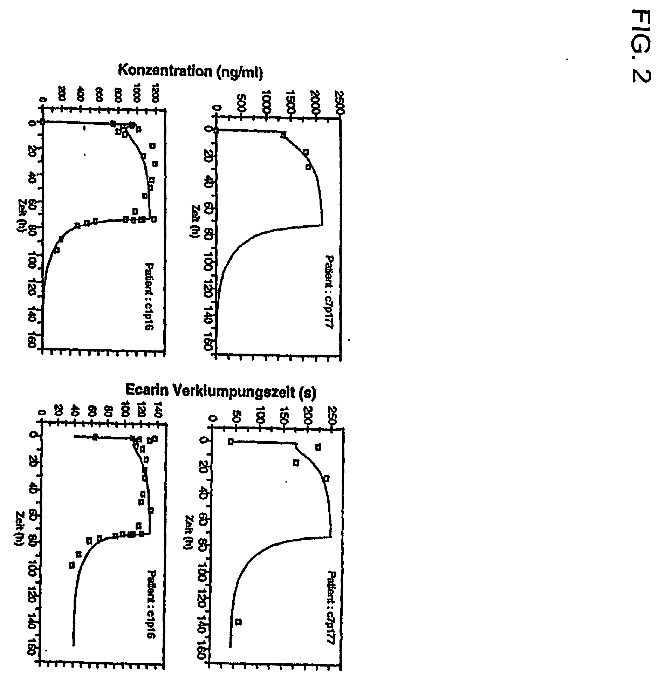

Der populationbasierte Ansatz ist insbesondere dann geeignet, wenn nur wenige Messungen bei einer großen Anzahl von Individuen erhoben wurden. In Abbildung 2 ist der beobachtete und geschätzte Zeitverlauf der PEG-Hirudin Konzentration im Plasma (links) und der 'Ecarin Verklumpungszeit' (rechts) bei zwei Patienten wiedergegeben. Falls genügend 'a priori' Wissen über die pk/pd Zusammenhänge aus vorherigen Studien verfügbar ist, ist eine Quantifizierung des pk/pd Verlaufes auch mit wenigen Beobachtungswerten möglich (oben). Aus Abb. 2 wird weiterhin der hohe lineare Zusammenhang von PEG-Hirudin Konzentration und Ecarin Gerinnungszeit deutlich. In solchen Situationen ist eine Bestimmung der individuellen pk/pd Parameter mit traditionellen Methoden der pk/pd Analyse nicht möglich.

Basierend auf dem populationsbasierten pk/pd Modell zur Beschreibung des Verhaltens von PEG-Hirudin nach unterschiedlicher Administrationsart und den gesammelten Erfahrungen aus den bisherigen Studien kann, falls erforderlich, eine Individualisierung der PEG-Hirudin Behandlung durchgeführt werden. Grundlage einer solchen individualisierten Therapie ist der Vergleich des Modellverhaltens mit dem klinischen Verhalten des Patienten.

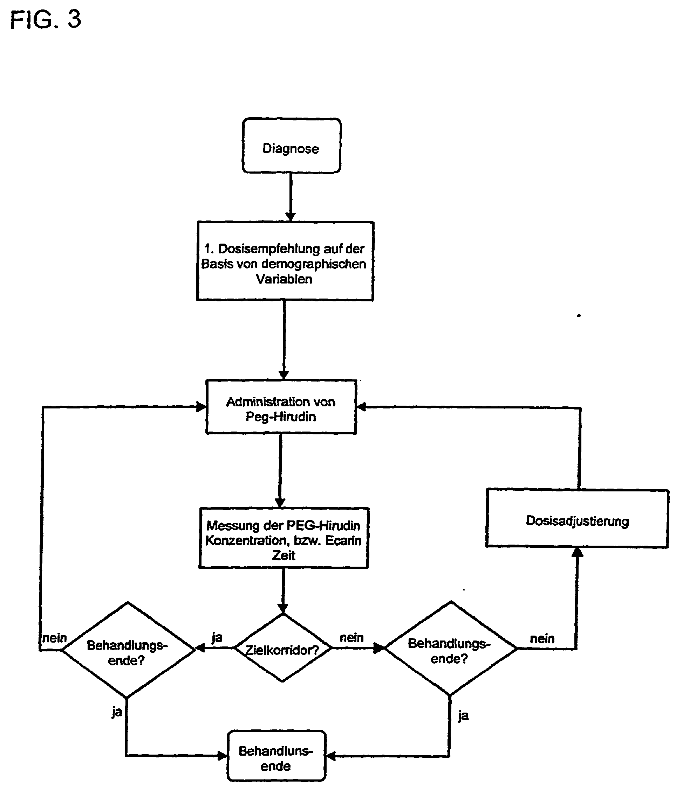

Abbildung 3 zeigt ein Ablaufschema bei der Behandlung von Patienten mit PEG-Hirudin.

Aus der bisherigen Erfahrung hat sich gezeigt daß eine 'Ecarin Gerinnungszeit' von über 250 s, bevorzugt von über 220 s keinen zusätzlichen Vorteil für den Patienten bringt, so daß diese als Obergrenze für eine eventuell notwendige Dosisadjustierung gelten kann. Gleichzeitig sollte eine 'Ecarin Gerinnungszeit' von 120 s, bevorzugt 150 s, im steady state nicht unterschritten werden.

Mit diesen aus der Klinik gewonnenen Werten wird daher ein "Zielkorridor für die Ecarin-Clotting-Time" vorgegeben, der eingehalten werden sollte. Das erfindungsgemäße Verfahren ermöglicht es, für den einzelnen Patienten sehr schnell zu bestimmen, welche Dosierung gewählt werden muß, damit bei der weiteren Behandlung diese Ober- und Untergrenzen eingehalten werden, so daß für den Patientenkreis kein, durch Hirudin bedingtes, Risiko besteht.

Beispiel:

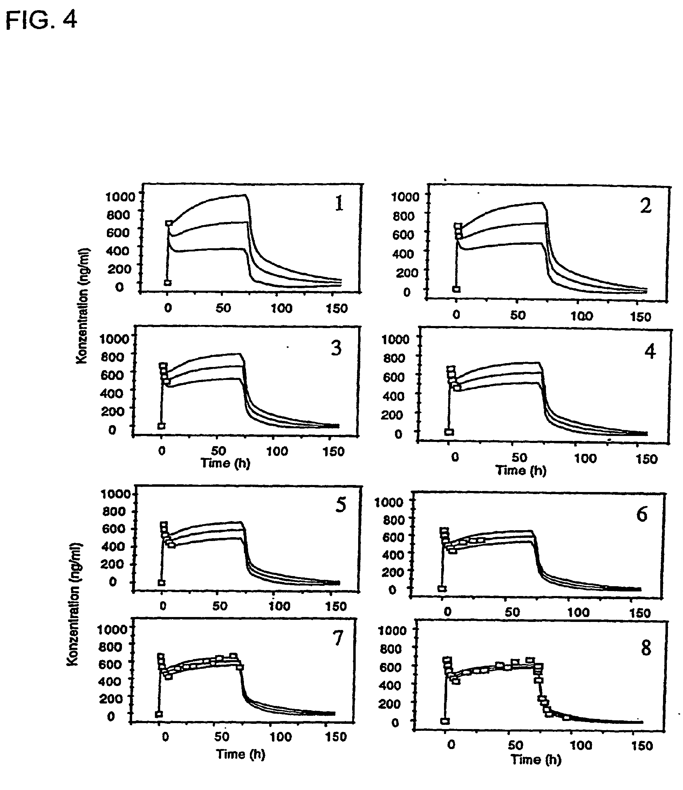

Abbildung 4 zeigt in einer Serie von Graphiken die Abhängigkeit des simulierten Plasma-Konzentrationsverlaufes von PEG-Hirudin eines repräsentativen klinischen Patienten von der Anzahl der in die Modellanpassung einbezogenen Meßzeitpunkten (Mittelwertsverlauf und 95 % Toleranzintervall). In der ersten Simulation wurde lediglich die erste Messung, die eine halbe Stunde nach Beginn der PEG-Hirudin Infusion erfolgte, in die Modellanpassung einbezogen und auf dieser Basis der weitere Verlauf der PEG-Hirudin Konzentration bei diesem Patienten prognostiziert. Alle anderen Messungen wurden bei dieser ersten Simulation vernachlässigt. Bei den weiteren Anpassungen wurden sukzessive mehr Meßzeitpunkte einbezogen.

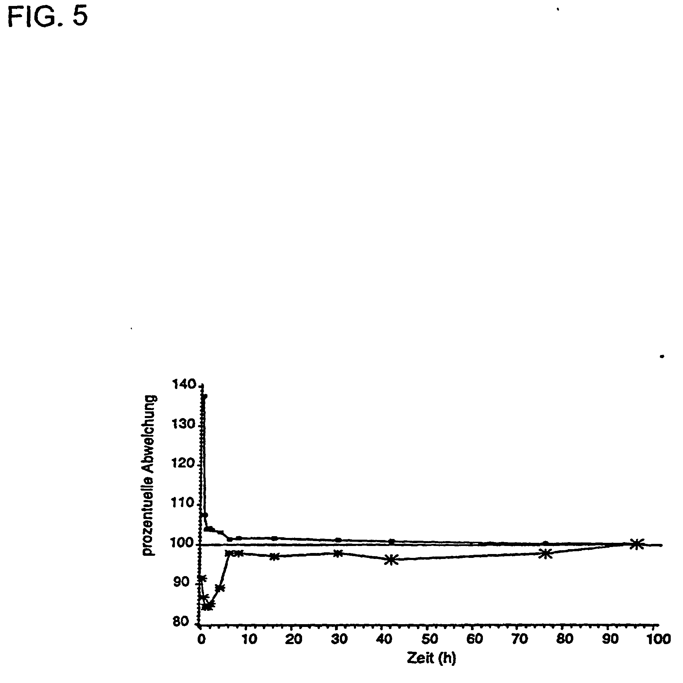

Abbildung 5 zeigt die prozentuale Abweichung der geschätzten Werte für die primären pharmakokinetischen Parameter 'scheinbares Verteilungsvolumen' (□) und 'Clearance' (*), von denjenigen Schätzwerten, die unter Einbeziehung aller Meßwerte dieses Patienten ermittelt wurden. Die geschätzten Werte für Verteilungsvolumen und Clearance sind nach ungefähr 4 Stunden (ca. 5 % der gesamten Infusionszeit) relativ konstant, so daß schon zu einem frühen Zeitpunkt während der Infusion, die 'steady state PEG-Hirudin Konzentration im Plasma' mit hoher Sicherheit prognostiziert und auf dieser Grundlage eine eventuell notwendige Dosiskorrektur vorgenommen werden kann.

Dies zeigt, das ein populationsbasierter pk/pd Modellansatz, zusammen mit wenigen frühzeitigen individuellen PEG-Hirudin oder Ecarin Verklumpungszeit Messungen als Grundlage einer eventuellen Dosisadaption bei Patienten dienen kann, um therapeutische Ziele mit PEG-Hirudin effektiver zu erreichen.

Zur Validierung des pk/pd Modelles wurden die beobachteten Daten von einer Gruppe von 26 Patienten (= Validierungspopulation) bei der Bestimmung der Modellparameter ausgeschlossen.

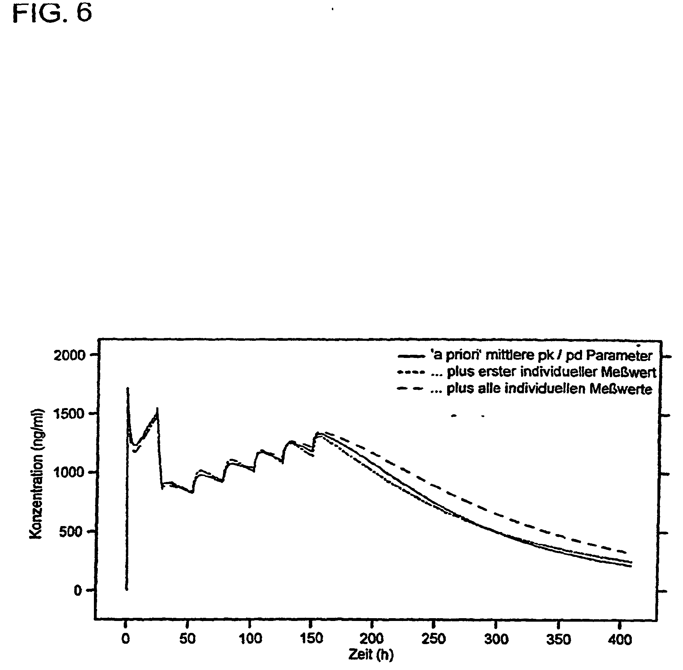

Abbildung 6 zeigt den geschätzten mittleren Verlauf der PEG-Hirudin Plasmakonzentration bei diesen Patienten auf der Basis der 'a priori' Werte der Modellparameter, d.h. mit den geschätzten mittleren Parameter, die für die Originalpopulation berechnet wurden (durchgezogene Linie). Abbildung 6 zeigt weiterhin die geschätzten 'a posteriori' Konzentrationsverläufe unter Einbeziehung des ersten (kurz gestrichelte Linie), bzw. aller individuellen PEG-Hirudin Konzentrationsmessungen (lang gestrichelte Linie) der Validierungspopulation.

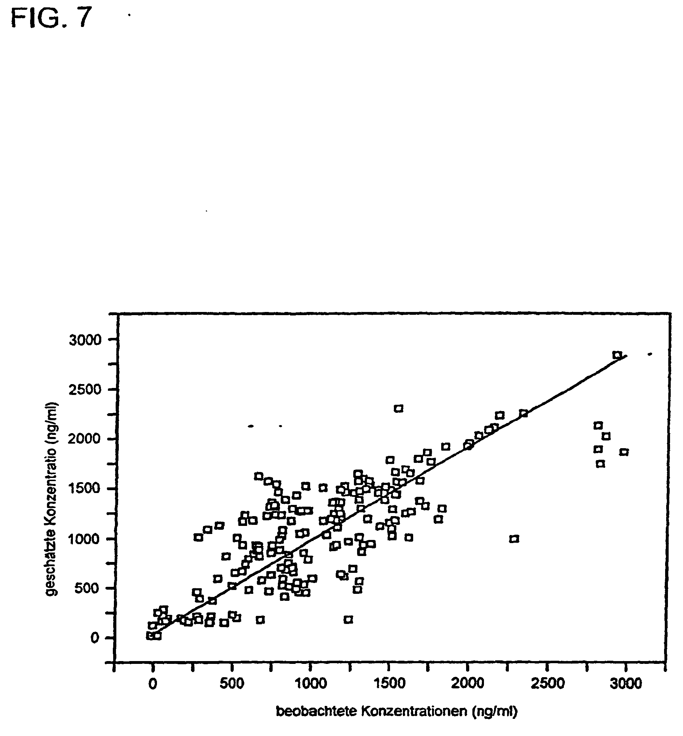

Abbildung 7 vergleicht die 'a posteriori', unter Einbeziehung des ersten individuellen Meßwertes, geschätzten individuellen PEG-Hirudin Konzentrationen mit den beobachteten Werten.

Die Übereinstimmung der geschätzten Konzentrationsverläufe mit den beobachteten Daten ist hoch, zumal bei der Validierungspopulation eine höhere subkutane Dosierung gegeben wurde als bei der Originalpopulation und von den demographischen Variablen der Validierungspopulation zum Zeitpunkt der Analyse lediglich das Körpergewicht bekannt war. Die individuellen Abweichungen lassen sich somit z.T. durch die bekannte, etwas nachlassende Bioverfügbarkeit mit zunehmender subkutan gegebener Dosis erklären.

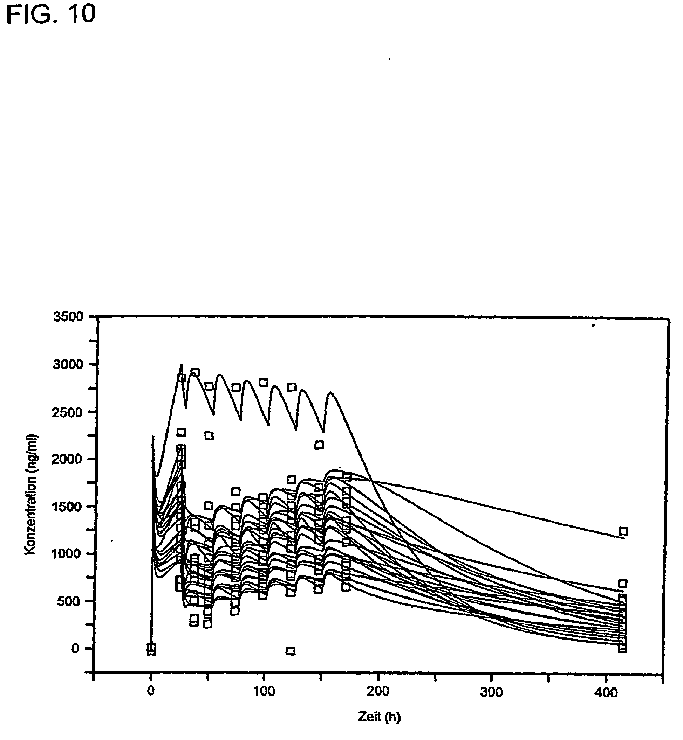

Abbildung 8 zeigt die 'a priori' geschätzten, individuellen Konzentrationsverläufe der Validierungspopulation, sowie die 'a posteriori' geschätzten individuellen Konzentrationsverläufe, unter Einbeziehung des ersten, bzw. aller individueller Konzentrationsmeßwerte (Abbildung 9 und 10).

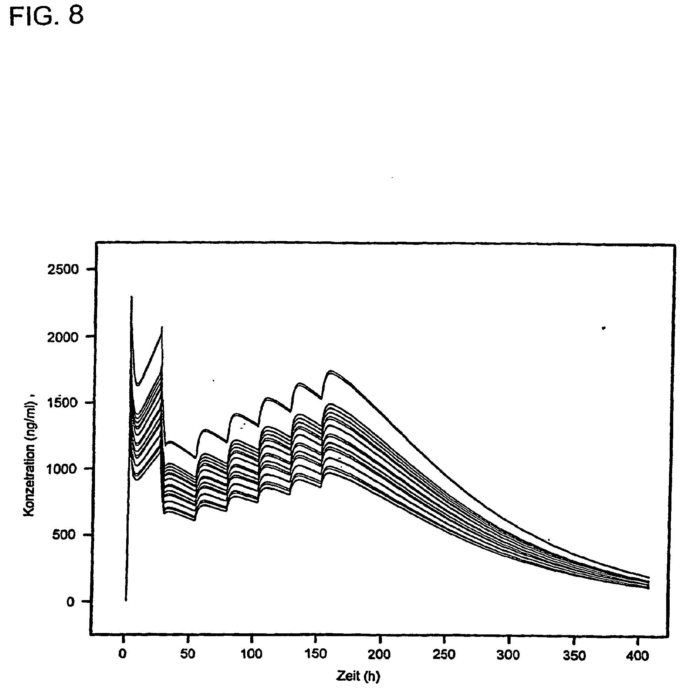

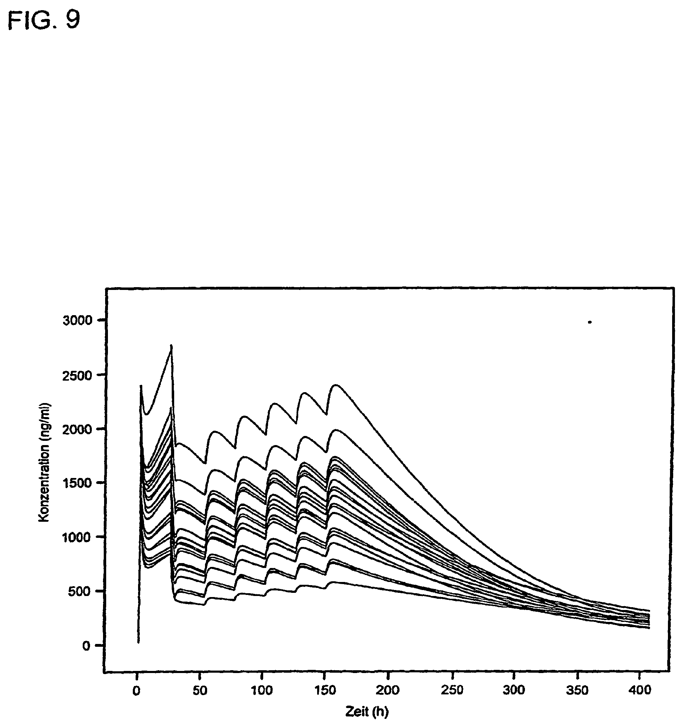

Die 'a priori' geschätzten individuellen Konzentrationsverläufe (Abbildung 8) zeigen bis auf einen Patient eine sehr gute Übereinstimmung mit den beobachteten Verläufen. Nach der ersten individuellen Messung (Abbildung 9) hätte dieser Patient, jedoch schon als 'Risikopatient' identifiziert werden können und entsprechend intensiver beobachtet, bzw. eine Dosisadjustierung vorgenommen werden können.

Zusammenfassend kann gesagt werden, daß die Brauchbarkeit des hier beschriebenen pk/pd Modelles zur Prognose des individuellen Verhaltens von PEG Hirudin gegeben ist und das pk/pd Modell beim Monitoring von PEG-Hirudin wertvolle Unterstützung geben kann.

**Patentansprüche**

1. Verfahren zur Ermittlung eines Dosierungsschemas für Hirudine oder orale Thrombininhibitoren, **dadurch gekennzeichnet, dass** man

   a) nach Zugabe einer Standarddosis des Hirudins oder des oralen Thrombininhibitors im Blut des Empfängers die Konzentration des Hirudins oder des oralen Thrombininhibitors mit dem Ecarin-Clotting-Time-Test bestimmt und

   b) aus dem Verhältnis von in Schritt a) zugegebener Dosis zu der ermittelten Ecarin-Clotting-Time mittels eines Computer-Programms, z.B. das Statistikprogrammpaket p-PHARM 1.4, SIMED, 1996, auf Basis eines pharmakokinetischen / pharmakodynamischen Modells, das durch die folgende Gleichung (6) dargestellt wird,

$$C_{ss\_i} = \frac{D\_i \times f\_i}{V\_i \times k_{el}\_i \times \tau\_i} \quad : \text{ (subkutane Injektion) (6)}$$

   wobei

   $C_{ss\_i}$ die individuelle 'steady state' Inhibitor Plasma Konzentration [ng/ml] ist;
   $D\_i$ die verabreichte Dosis [mg] im Applikationsintervall ist;
   $f\_i$ die Bioverfügbarkeit der subkutanen Administration [ohne Dimension] ist;
   $V\_i$ das scheinbare Verteilungsvolumen [l] von Hirudin im Patienten ist;
   $k_{el}\_i$ die scheinbare Eliminationsrate [1/h] von Hirudin im Patienten ist; und
   $\tau\_i$ das Applikationsintervall [h] ist,

   die Dosierung des weiterhin zuzugebenden Hirudins oder oralen Thrombininhibitors ermittelt.

2. Verfahren nach Anspruch 1, **dadurch gekennzeichnet, dass** als Hirudin natürliches Hirudin, rekombinantes Hirudin oder an organische Träger kovalent gebundenes Hirudin eingesetzt wird.

3. Verfahren nach Anspruch 2, **dadurch gekennzeichnet, dass** als Hirudin an Polyethylenglykol gebundenes Hirudin verwendet wird.

4. Verfahren nach Anspruch 1, **dadurch gekennzeichnet, dass** als orale Thrombininhibitoren Verbindungen eingesetzt werden, die als Struktureinheit einen Phenyl- oder heterocyclischen Ring mit einer Amidingruppe als Substituent aufweisen.

**Claims**

1. Method for establishing a dosage plan for hirudins or oral thrombin inhibitors, **characterized in that**

   a) after administration of a standard dose of the hirudin or of the oral thrombin inhibitor, the concentration of the hirudin or of the oral thrombin inhibitor in the blood of the recipient is determined using the ecarin clotting time test and

   b) from the ratio of the dose administered in step a) to the ecarin clotting time determined, the dosage of the hirudin or oral thrombin inhibitor for continued administration is determined by means of a computer program, e.g. the p-PHARM 1.4, SIMED, 1996 statistics program package, on the basis of a pharmacokinetic/pharmacodynamic model which is represented by the following equation (6)

   $$C_{ss\_i} = \frac{D\_i \times f\_i}{V\_i \times k_{el}\_i \times \tau\_i} \quad : \text{(subcutaneous injection) (6)}$$

   wherein

   $C_{ss\_i}$ is the individual "steady state" inhibitor plasma concentration [ng/ml] ;
   $D\_i$ is the dose administered [mg] in the administration interval;
   $f\_i$ is the bioavailability of the subcutaneous administration [dimensionless];
   $V\_i$ is the apparent distribution volume [1] of hirudin in the patient;
   $k_{el}\_i$ is the apparent elimination rate [1/h] of hirudin in the patient; and
   $\tau\_i$ is the administration interval [h].

2. Method according to claim 1, **characterized in that** natural hirudin, recombinant hirudin or hirudin covalently bonded to organic carriers is employed as the hirudin.

3. Method according to claim 2, **characterized in that** hirudin bonded to polyethylene glycol is used as the hirudin.

4. Method according to claim 1, **characterized in that** compounds which contain, as a structural unit, a phenyl or heterocyclic ring having an amidine group as a substituent are employed as oral thrombin inhibitors.

**Revendications**

1. Procédé de détermination d'un schéma posologique pour des hirudines ou des inhibiteurs oraux de la thrombine, **caractérisé en ce que** l'on

   a) détermine après administration d'une dose standard d'hirudine ou d'inhibiteur oral de la thrombine la concentration d'hirudine ou d'inhibiteur oral de thrombine dans le sang du receveur grâce au test du temps d'écarine (Ecarin-Clotting-Time) et que l'on

   b) établit la posologie de l'hirudine ou de l'inhibiteur oral de la thrombine à administrer ensuite d'après le rapport entre la dose administrée à l'étape a) et le temps d'écarine déterminé, à l'aide d'un programme informatique, par exemple le pack de programmes statistiques p-PHARM 1.4, SIMED, 1996, sur la base d'un modèle pharmacocinétique/pharmacodynamique, représenté par l'équation suivante (6) :

   $$C_{ss\_i} = \frac{D\_i \times f\_i}{V\_i \times k_{el}\_i \times \tau\_i} \quad : \text{(injection sous-cutanée) (6)}$$

   où

$C_{ss\_i}$ est la concentration plasmatique individuelle à l'état d'équilibre en [ng/ml] de l'inhibiteur ;

$D\_i$ est la dose administrée en [mg] dans l'intervalle d'administration ;

$f\_i$ est la biodisponibilité pour l'administration sous-cutanée [sans dimension] ;

$V\_i$ est le volume apparent de distribution en [1] chez le patient ;

$k_{el\_i}$ est la vitesse apparente d'élimination en [1/h] de l'hirudine chez le patient ; et

$\tau\_i$ est l'intervalle d'administration en [h].

2. Procédé selon la revendication 1, **caractérisé en ce que** l'on utilise comme hirudine de l'hirudine naturelle, de l'hirudine recombinante ou de l'hirudine liée par covalence à des supports organiques.

3. Procédé selon la revendication 2, **caractérisé en ce que** l'on utilise comme hirudine de l'hirudine liée par covalence à du polyéthylèneglycol.

4. Procédé selon la revendication 1, **caractérisé en ce que** l'on utilise comme inhibiteurs oraux de la thrombine des composés qui comprennent comme unité structurelle un noyau phényle ou un noyau hétérocyclique avec un groupe amidine comme substituant.

# FIG. 1

$$E(t) = E_{min} + A * C_p(t)$$

Effekt
E

iv.
(Bolus oder Infusion)

sc. → 

Gewebe
$S_1$

$k_{abs}$
$T_{lag}, f$

zentrales
Kompartiment
$C_p$
V

$k_{12}$

Gewebe
$S_2$

$k_{21}$

$k_{el} = (k_r + ...)$

FIG. 2

## FIG. 3

```
                    ┌─────────────┐
                    │  Diagnose   │
                    └──────┬──────┘
                           │
                           ▼
              ┌──────────────────────────┐
              │ 1. Dosisempfehlung auf der │
              │ Basis von demographischen  │
              │        Variablen           │
              └─────────────┬──────────────┘
                            │
                            ▼
              ┌──────────────────────────┐
         ┌───▶│    Administration von     │◀───┐
         │    │       Peg-Hirudin         │    │
         │    └─────────────┬─────────────┘    │
         │                  │                  │
         │                  ▼           ┌──────────────────┐
         │    ┌──────────────────────┐  │  Dosisadjustierung │
         │    │ Messung der PEG-Hirudin│  └─────────▲────────┘
         │    │ Konzentration, bzw. Ecarin         │
         │    │        Zeit          │             │
         │    └───────────┬──────────┘             │
         │                │                        │
```

Behandlungs-ende?   nein   Zielkorridor?   ja / nein   Behandlungs-ende?   nein

ja

Behandluns-ende

ja

FIG. 4

# FIG. 5

# FIG. 6

# FIG. 7

beobachtete Konzentrationen (ng/ml)

FIG. 8

FIG. 9

FIG. 10